# EUROPEAN PATENT SPECIFICATION

(11) **EP 4 370 054 B1**
(45) Date of publication and mention of the grant of the patent: **22.04.2026**
(21) Application number: 22765266.6
(22) Date of filing: 13.07.2022
(51) Int. Cl.: A61B 34/20, A61B 17/00

(54) **DISTAL TIP TRACKING AND MAPPING**
VERFOLGUNG UND ABBILDUNG DISTALER SPITZEN
SUIVI ET CARTOGRAPHIE DE POINTE DISTALE

(30) Priority: 15.07.2021 US 202163221995 P
(43) Date of publication of application: 22.05.2024
(60) Divisional application: 26164826.5
(73) Proprietor: Boston Scientific Scimed Inc., Maple Grove, Minnesota 55311 (US)
(72) Inventor: LIM, Kian S., Shrewsbury, Massachusetts 01545 (US); RAUNIYAR, Niraj Prasad, Plymouth, Minnesota 55446 (US); CHEN, Longquan, Andover, Massachusetts 01810 (US); FOLEY, Sarah, Andover, Massachusetts 01810 (US)
(74) Representative: Pfenning, Meinig & Partner mbB
(86) International application number: PCT/US2022/037028
(87) International publication number: WO 2023/287927

(56) References cited:
- WO-A1-2015/061756
- WO-A1-2016/164311
- US-A1- 2019 053 860
- US-A1- 2021 196 398

## Description

### CROSS REFERENCE TO RELATED APPLICATIONS

This application claims the benefit of and priority to U.S. Provisional Patent Application Serial No. 63/221,995 filed on July 15, 2021.

### TECHNICAL FIELD

The present disclosure relates to navigation, mapping, and tracking during endoscopic procedures. More particularly, the present disclosure relates to the use of an accelerometer for navigation, mapping, and tracking during endoscopic procedures.

### BACKGROUND

During endoscopic procedures, there is a need to know the position of the scope tip. Currently, the position of the scope as well as other tools are tracked using fluoroscopy. However, when using fluoroscopy, there is a risk of prolonged exposure to radiation for the patient, physician, and operating room staff. Conventional electromagnetic sensors may be large and/or expensive. Conventional electromagnetic sensors may also require special conditions, such as, but not limited to, ferrite free environments and/or expensive capital. Further, even when using fluoroscopy and/or active computerized tomography (CT) are used, it may be impractical to receive real time data, such as roll, pitch, and positioning data of the endoscope and/or other tools. It may be desirable to provide systems and methods for navigation, mapping, and tracking of an endoscope which allow for the gathering of real-time data as well as a reduction in the exposure to radiation.

Document US 2019/053860 A1 describes techniques to facilitate augmented reality-assisted surgery. A method is described that includes receiving, by a first device including a processor, image data associated with an external portion of a tool located within a body of a patient, wherein the image data includes first information indicative of a first fiducial marker on the external portion of the tool. The method also includes determining one or more relative positions of an internal portion of the tool within the body relative to one or more anatomical structures of the body based on the image data and a defined configuration of the tool. The method also includes generating one or more representations of the tool within the body relative to the one or more anatomical structures based on the one or more relative positions and the defined configuration of the tool.

Document WO 2015/061756 A1 describes an endolumenal robotic system that provides the surgeon with the ability to drive a robotically-driven endoscopic device to a desired anatomical position in a patient and that includes a digital camera mounted on the endoscopic device.

Document US 20211/96398 A1 describes methods of positioning a surgical instrument that involves advancing a first medical instrument to a treatment site of a patient, the first medical instrument comprising a camera, recording a target position associated with a target anatomical feature at the treatment site, generating a first image of the treatment site using the camera of the first medical instrument, identifying the target anatomical feature in the first image using a pretrained neural network, and adjusting the target position based at least in part on a position of the identified target anatomical feature in the first image.

### BRIEF SUMMARY

This disclosure provides design, material, manufacturing method, and use alternatives for medical devices. The invention is defined in claim 1. Any embodiment that is inconsistent with the subject-matter of claim 1 is not part of the invention. Methods for treatment the human or animal body by surgery are also not part of the invention.

In a first example, a method for determining and mapping a location of a distal end region of an elongate shaft may comprise obtaining data from an accelerometer, the accelerometer located in the elongate shaft adjacent a distal end thereof, determining a length of the elongate shaft inserted into a body from a reference point, merging the accelerometer data and the length of the elongate shaft to localize the distal end region of the elongate shaft, reconstructing a line of travel of the medical device within the body, and superimposing the reconstructed line of travel over an image of an anatomy of the patient.

Alternatively or additionally to any of the examples above, in another example, the method may further comprise obtaining an image from a camera positioned at the distal end of the elongate shaft.

Alternatively or additionally to any of the examples above, in another example, the method may further comprise detecting feature points from the image.

Alternatively or additionally to any of the examples above, in another example, the method may further comprise merging the feature points and the localized distal end region of the elongate shaft.

Alternatively or additionally to any of the examples above, in another example, the image of the anatomy may be a 3-dimensional (3D) reconstruction of the anatomy adjacent to the distal end region.

Alternatively or additionally to any of the examples above, in another example, the image of the anatomy may be a 3-dimensional CT scan.

Alternatively or additionally to any of the examples above, in another example, obtaining the data from an accelerometer and determining the length of the elongate shaft inserted into the body may be performed with the distal end of the elongate shaft at a same location.

Alternatively or additionally to any of the examples above, in another example, obtaining data from an accelerometer may comprise obtaining accelerometer data from a plurality of different locations of the distal end of the elongate shaft.

Alternatively or additionally to any of the examples above, in another example, determining the length of the elongate shaft inserted into the body may comprise obtaining a length from a plurality of different locations of the distal end of the elongate shaft.

Alternatively or additionally to any of the examples above, in another example, obtaining an image may comprise obtaining an image from a plurality of different locations of the distal end of the elongate shaft.

In another example, a system for determining a position of a medical device in a body may comprise an elongate shaft extending from a proximal end to a distal end, an accelerometer located in the elongate shaft adjacent to the distal end thereof, a plurality of demarcations formed on an outer surface of the elongate shaft, the demarcations longitudinally spaced at predefined intervals along a length of the elongate shaft, a first optic sensor and a second optic sensor, the first and second optic sensors configured to be positioned adjacent to the body and longitudinally spaced apart, and a computing device operably coupled to the accelerometer, the first optic sensor and the second optic sensor.

Alternatively or additionally to any of the examples above, in another example, the first and second optic sensors may be configured to detect and count the plurality of demarcations as the elongate shaft is advanced into the body.

Alternatively or additionally to any of the examples above, in another example, the first and second optic sensors may be configured to detect a direction of travel of the elongate shaft.

Alternatively or additionally to any of the examples above, in another example, the computing device may be configured to determine a length of the elongate shaft that has been advanced into the body from a reference point based on the detection and counting of the plurality of demarcations.

Alternatively or additionally to any of the examples above, in another example, the system may further comprise a gyro sensor.

In another example, a method for tracking a location of a medical device may comprise advancing a medical device within a body of a patient, receiving data from an accelerometer, the accelerometer positioned adjacent a distal end of an elongate shaft of the medical device, determining an insertion length of the elongate shaft, reconstructing a line of travel of the medical device within the body, superimposing the reconstructed line of travel over an image of an anatomy of the patient, and displaying the image of the anatomy with the superimposed line of travel on a user interface.

Alternatively or additionally to any of the examples above, in another example, the image of the anatomy may be a 3-dimensional CT scan.

Alternatively or additionally to any of the examples above, in another example, the method may further comprise positioning virtual markers on the image of the anatomy with the superimposed line of travel.

Alternatively or additionally to any of the examples above, in another example, the virtual markers may be placed through actuation of a button on a handle of the medical device.

Alternatively or additionally to any of the examples above, in another example, the method may further comprise receiving data from a gyro sensor,

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
FIG. 1 is a schematic illustration of an example endoscopic system;
FIG. 2 is a perspective view of a portion of the distal end region of the elongate shaft of the endoscopic system of FIG. 1;
FIG. 3 is a side view of the distal end region of the elongate shaft of the endoscopic system of FIG. 1;
FIG. 4 is a schematic block diagram of an illustrative computing device that may be used with the endoscopic system of FIG. 1;
FIG. 5 is an illustrative flow chart of a method for determining the location of the distal end region of an elongate shaft and rendering the system on a display;
FIG. 6 is a schematic representation of an elongate shaft moving through the body and obtaining data;
FIG. 7 is a flow chart of another illustrative method for tracking a location of an endoscopic system;
FIG. 8 illustrates a line of travel of an elongate shaft over a 3D scan of the intrarenal area; and
FIG. 9 is a flow chart of another illustrative method for tracking a location of an endoscopic system.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

Systems and methods for the navigation, mapping, and tracking of an endoscope and/or various tools used therewith are described herein. During a medical procedure (e.g., an endoscopic procedure, ureteroscopic procedure, etc.), it is desirable for the physician to know the location of the distal tip of the endoscope, ureteroscope, or other device or tool to help the physician navigate complex anatomy. To help the physician navigate in the anatomy, an accelerometer chip may be embedded in the distal tip of an elongate shaft of a device. The accelerometer may provide the direction of movement and/or acceleration of the chip in the X, Y, and Z directions, and thus the direction movement and/or acceleration of the distal tip in the X, Y, and Z directions. Together with determining a length of insertion and/or retraction of the shaft of the device, the movement of the device in 3-dimensional (3-D) space can be computed. While the present systems and methods are described with respect to an endoscope, it should be understood the present disclosure is not limited to such a device. For example, the systems and methods described herein may be used with other medical devices in which navigation, mapping, and/or tracking are utilized including, but not limited to ureteroscopes, colonoscopes, nephroscopes, catheters, intravascular devices, etc.

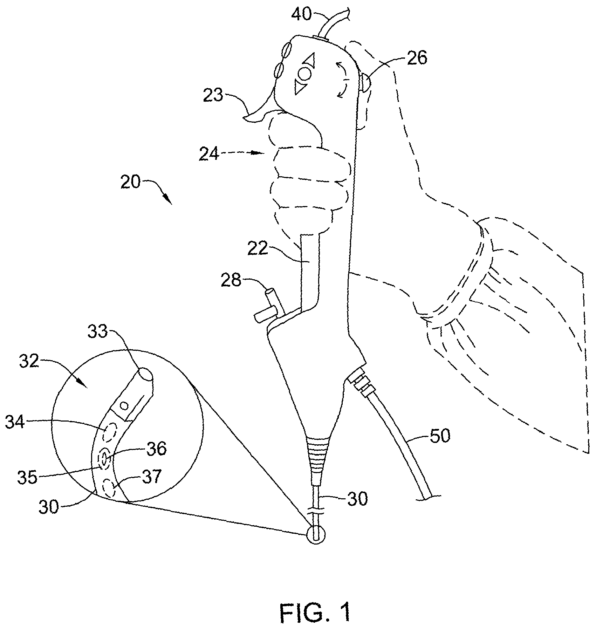

FIG. 1 illustrates an example medical device 20, such as, but not limited to an endoscopic system, that may be used in conjunction with other aspects of the disclosure. In some cases, the endoscopic system 20 may be configured to be used in conjunction with a fluid management system (not shown), although this is not required. In some embodiments, the endoscopic system 20 may be a ureteroscope such as a LithoVue^{™} scope. However, other medical devices, such as another endoscope, may be used in addition to or in place of a ureteroscope. The endoscopic system 20 may include a handle 22 and an elongate shaft 30 extending distally from the handle 22. The endoscopic system 20 may be configured to deliver fluid from the fluid management system to a treatment site via the elongate shaft 30, which may be configured to access the treatment site within the patient. In some embodiments, a fluid source and/or an inflow pump may be in fluid communication with the endoscopic system 20 and/or the elongate shaft 30. The elongate shaft 30 may include one or more working lumens for receiving a flow of fluid or other medical devices therethrough. The endoscopic system 20 may be connected to the fluid management system via one or more supply line(s) 40. While not explicitly shown, the elongate shaft 30 may include one or more working lumens for receiving the fluid and/or other medical devices.

In some embodiments, the endoscopic system 20 may be in electronic communication with a workstation via a wired connection or data transmission cable 50. In some embodiments, the workstation may include a touch panel computer, an interface box for receiving the wired connection 50, a cart, and a power supply, among other features. In some embodiments, the electronic device to which the wired connection 50 is connected may have functionality for recognizing and exchanging data with other endoscopic accessories. Other configurations are also contemplated. In some embodiments, the interface box may be configured with a wired or wireless communication connection with a controller of the fluid management system. The touch panel computer may include at least a display screen and an image processor. In some embodiments, the workstation may be a multi-use component (e.g., used for more than one procedure) while the endoscopic system 20 may be a single use device, although this is not required. In some embodiments, the workstation may be omitted and the endoscopic system 20 may be electronically coupled directly to the controller of the fluid management system. Additionally, or alternatively, a microprocessor may be embedded in the handle 22 of the endoscopic system 20. In some cases, the wired connection may be configured to transmit data and/or image signals from one or more components of the endoscopic system 20. It is contemplated that the elongate shaft 30 may include one or more working lumens within which the data transmission cable 50 (e.g., fiber optic cable, optic cable, connector, wire, etc.) may extend.

As shown in FIG. 1, the endoscopic system 20 may include one or more sensors proximate a distal end region 32 of the elongate shaft 30. For example, the endoscopic system 20 may include a pressure sensor 34 at a distal tip of the elongate shaft 30 to measure intracavity pressure within the treatment site. The endoscopic system 20 may also include other sensors such as, for example, a temperature sensor 35, an accelerometer 36 (e.g., a position sensor), and/or another sensor. Other sensors 37 may include, but are not limited to, a Fiber Bragg grating optical fiber to detect stresses, a gyro sensor for detecting angular velocity, shape sensing sensors, etc. In an illustrative embodiment, the distal end region 32 of the elongate shaft 30 and/or the endoscopic system 20 may also include at least one camera 33 to provide a visual feed to the user on a display screen. In another embodiment, the at least one camera 33 may include two cameras having different communications requirements or protocols so that different information may be relayed to the user by each camera. When so provided, the user may switch back and forth between cameras at will through a touch screen interface and/or a touch panel computer. When so provided, the camera 33 and/or sensors 34, 35, 36, 37 may be communicatively coupled to the workstation, or other computing device via a transmission cable, such as cable 50. Alternatively, or additionally, the camera 33 and/or sensors 34, 35, 36, 37 may be communicatively coupled to the workstation, or other computing device via a wireless connection.

In some embodiments, the location of the distal end region 32 of the elongate shaft 30 may be tracked during use. For example, as will be described in more detail herein, the accelerometer 36 may provide the direction of the movement and/or acceleration of the chip which may be used to determine a location of the distal end region 32. In some cases, the accelerometer 36 may be used in combination with a CT scan, or other imaging capability. The workstation and/or control unit may communicate to determine the position of the accelerometer 36 relative to the patient.

The endoscopic system 20 includes the handle 22 coupled to a proximal end of the elongate shaft 30. The handle 22 may have an optional fluid flow on/off switch 23, which allows the user to control when fluid is flowing through the endoscopic system 20 and into the treatment site. The handle 22 may further include other buttons 24, 26 that perform other various functions. For example, in some embodiments, the handle 22 may include buttons to control the temperature of the fluid, fluid flow rate, activation of another medical device, etc. It will be understood that while the exemplary embodiment describes a ureteroscope, the features detailed above may also be directly integrated into another endoscope (e.g., a cystoscope, a nephroscope, a hysteroscope), or virtually any device with an image capability. In some embodiments, the endoscopic system 20 may also include a drainage port 28 which may be connected to a drainage system (not shown). Some illustrative drainage systems are described in commonly assigned U.S. Patent Application Publication No. 2018/0361055, titled AUTOMATED FLUID MANAGEMENT SYSTEM.

Briefly, the fluid management system may include an inflow pump configured to pump and/or transfer fluid from a fluid supply source (e.g., a fluid bag, etc.) to the medical device 20 and/or a treatment site within a patient at a fluid flow rate. In some cases, the fluid may pass through a fluid warming system for heating fluid to be delivered to the patient via the one or more supply line(s) prior to entering the endoscopic system 20. In some embodiments, the fluid management system may be one that may be used in an endoscopic procedure, such as flexible ureteroscopy (fURS) procedures (e.g., ureteroscopy, percutaneous nephrolithotomy (PCNL), benign prostatic hyperplasia (BPH), transurethral resection of the prostate (TURP), etc.), gynecology, and other endoscopic procedures. Some illustrative fluid management systems and fluid warming systems are described in described in commonly assigned U.S. Patent Application Publication No. 2018/0361055, titled AUTOMATED FLUID MANAGEMENT SYSTEM.

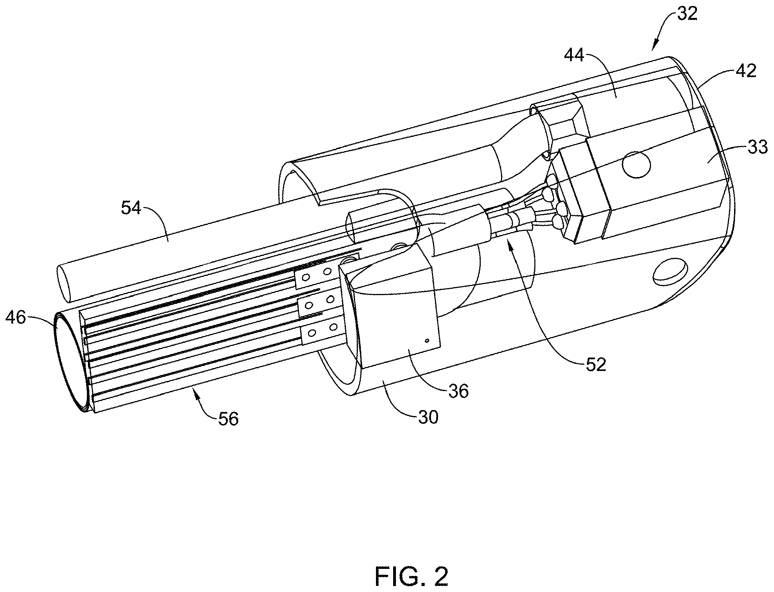

FIG. 2 illustrates a perspective view of a portion of the distal end region 32 of the elongate shaft 30 of the endoscopic system 20. To better illustrate the configuration of some components internal to the elongate shaft 30, a portion of the elongate shaft 30 is not shown while a more distal portion of the elongate shaft 30 is shown in transparency. As described above, the endoscopic system 20 may include a camera 33 positioned at or adjacent to a distal end 42 of the elongate shaft 30. The camera 33 may be communicatively coupled to a workstation, or other processing device, via one or more data transmission cables 52. The data transmissions cables 52 may extend proximally from the camera 33 through the elongate shaft 30 to the handle 22. In some cases, a light source 44 may also be provided at or adjacent to the distal end 42 of the elongate shaft 30. The light source 44 may be coupled to a power source via a cable 54. The cable 54 may be extend proximally from the light source 44 through the elongate shaft 30 to the handle 22. A working channel 46 may extend from a proximal end (not explicitly shown) of the elongate shaft 30 to the distal end 42 thereof. The working channel 46 may allow another device or tool to be advanced through the elongate shaft 30 and exit the distal end 42 thereof.

The distal end region 32 of the elongate shaft 30 may further include an accelerometer 36. The accelerometer 36 may be a multi-axis accelerometer, such as, but not limited to a 3-axis accelerometer or a 6-axis accelerometer. For example, the accelerometer 36 may measure acceleration in the X, Y, and Z planes. Additionally or alternatively, the accelerometer 36 may measure acceleration in the yaw, pitch, and roll axes. The accelerometer 36 may be positioned a predetermined or known distance from the distal end 42 of the elongate shaft 30 in order to determine a location of the distal tip. The accelerometer 36 may have a size that allows it to be disposed within or embedded within the elongate shaft 30. It is contemplated that the accelerometer 36 need not be positioned in a particular orientation. For example, the accelerometer 36 is not necessarily positioned such that it is oriented towards the distal end 42.

The accelerometer 36 may be communicatively coupled to a workstation, or other processing device, via one or more data transmission cables 56. The data transmissions cables 56 may extend proximally from the accelerometer 36 through the elongate shaft 30 to the handle 22. In the illustrated embodiment, the accelerometer 36 may include eight connector points and therefore eight cables 56 may extend from the accelerometer 36 through the elongate shaft 30 to the handle 22. However, fewer than eight or more than eight connector points and/or cables 56 may be provided, as desired.

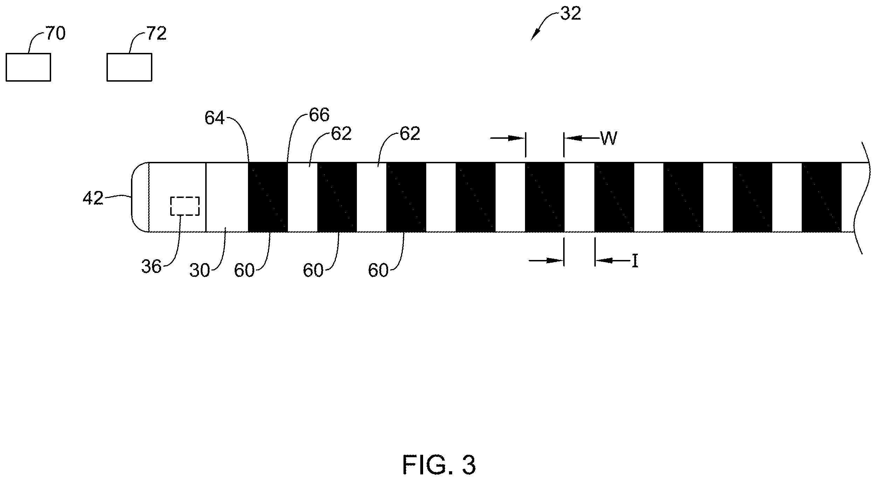

It is contemplated that the direction of movement and/or acceleration of the accelerometer 36 in the X, Y, Z direction as provided by the accelerometer 36 can be combined with a length of insertion and/or retraction of the elongate shaft 30 to compute the movement of the endoscopic system 20. FIG. 3 is a side view of the distal end region 32 of the elongate shaft 30. The elongate shaft 30 may include a plurality of longitudinally spaced demarcations 60 (e.g., spaced apart dark lines, spaced apart surface texture, spaced apart indicia, etc. extending about a perimeter of the elongate shaft 30 on an outer surface thereof. The longitudinally spaced demarcations 60 may extend along an entire length of the elongate shaft 30 or one or more portions thereof, such as the distal end region 32. It is contemplated that the demarcations 60 may include other structures or features. For example, the demarcations 60 may include etchings that are laser readable. Each of the demarcations 60 may extend circumferentially continuously around the entire perimeter of the elongate shaft 30, or each of the demarcations 60 may extend discontinuously around the perimeter of the elongate shaft 30. The demarcations 60 may be longitudinally spaced apart at predefined intervals I and have a predefined width W. The demarcations 60 may be separated by a region 62 free from markings also having a predefined width. The width of the region 62 free from markings may be the same as the predefined interval I. It is contemplated that each demarcation 60 may have the same predefined width W and the same predefined spacing interval I such that the demarcations 60 may be utilized to determine a length of the elongate shaft 30 that has been inserted or retracted from the body. The predefined width W and the predefined interval I may have a same length or differing lengths, as desired.

The demarcations 60 may be used in conjunction with one or more sensors to determine the insertion length of the elongate shaft 30, and thus the distance the distal end region 32 of the elongate shaft has traveled from some reference point (e.g., the entry point into the patient). The demarcations 60, in conjunction with the one or more sensors may also determine the direction of movement of the elongate shaft, either distal advancement into the body of the patient or proximal retraction out of the body of the patient. For example, the distal end 64 of the demarcation 60 may create a first output and the proximal end 66 of the demarcation 60 may create a second output. At least one sensor, or two or more sensors 70, 72 (schematically illustrated in FIG. 3) may be at the entry point to the body. For example, the sensors 70, 72 may be positioned in a housing at the entry point into the patient's body. The elongate shaft 30 may pass through an aperture of the housing and into the patient's body at the entry point, with the sensors 70, 72 facing the elongate shaft 30 passing through the aperture of the housing. In some cases, the sensors 70, 72 may be optic sensors configured to detect the demarcations 60. Alternatively, or additionally to, the sensors 70, 72, a camera may be used to detect two or more demarcations 60, such as spaced apart demarcations, along the elongate shaft 30. These sensors 70, 72 may read the first and second outputs to determine the distal tip displacement from the entry point to the body. For example, the two or more sensors 70, 72 may be laterally spaced such that the sensors 70, 72 receive the first output at different times and the second output at different times. For example, the output signals received at each sensor 70, 72 may be out of phase with one another. This may allow the sensors 70, 72 to determine both distance (by counting the demarcations 60) and direction (by comparing output signals). For example, when the elongate shaft 30 is distally advanced, the sensors 70, 72 may receive output signals which are opposite. When the elongate shaft 30 is proximally retracted, the sensors 70, 72 may receive output signals which are the same. The workstation or controller can be programmed to read the output signals from the sensors 70, 72 and determine both a distance of travel and a direction of travel (e.g., distal advancement into the body or proximal retraction out of the body) of the elongate shaft 30.

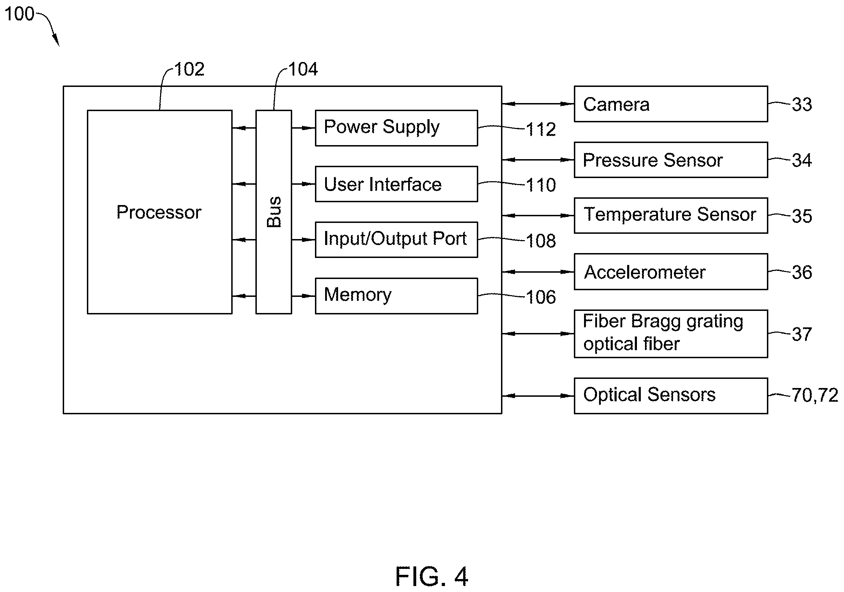

FIG. 4 is a schematic block diagram of an illustrative computing device 100 that may be used to control the endoscopic system 20 and/or process data obtained therefrom. It is contemplated that the computing device 100 may be any computing device suitable for receiving inputs and providing outputs to the various components of the endoscopic system. Examples of computing devices include specialized computing devices or general-purpose computing devices such "control units," "control assemblies," "workstations," "servers," "hand-held devices," "controllers," and the like.

The computing device 100 may include a bus 104 that, directly and/or indirectly, couples the following devices: a processing unit 102, a memory 106, an input/output (I/O) port 108, a user interface 110, and a power supply 112. Any number of additional components, different components, and/or combinations of components may also be included in or connected to the computing device. The memory 106 may be in communication with the processor 102. The memory 106 may be used to store any desired information such as, but not limited to, control algorithms, configuration protocols, set points, and the like. In some embodiments, the memory 106 may include specific programs or modules configured to determine a position and/or orientation of the distal end region 32 of the elongate shaft 30 and/or generate an image of the endoscopic system 20. The memory 106 may be any suitable type of storage device including, but not limited to, RAM, ROM, EPROM, flash memory, a hard drive, and/or the like. In some cases, the processor 102 may store information within the memory 106 and may subsequently retrieve the stored information from the memory 106. In embodiments, the memory 106 stores computer-executable instructions for causing the processor 102 to implement aspects of embodiments of system components discussed herein and/or to perform aspects of embodiments of methods and procedures discussed herein.

The computer-executable instructions may include, for example, computer code, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

The input/output port (I/O port) 108 may have a number of wire terminals for receiving one or more signals from the camera 33, sensors 34, 35, 36, 37, 70, 72 and/or system components and/or for providing one or more signals to the system components. In some cases, the I/O port 108 may communicate with one or more components of the endoscopic system 20, including, but not limited to, the camera 33 and/or sensors 34, 35, 36, 37, 70, 72. The computing device 100 may have any number of wire terminals for accepting a connection from one or more components of the system 10. However, how many wire terminals are utilized and which terminals are wired is dependent upon the particular configuration of the endoscopic system 20. Different systems 20 having different components and/or types of components may have different wiring configurations. In some cases, the I/O port 108 may be configured to receive wireless signals from the camera 33, sensors 34, 35, 36, 37, 70, 72 and/or one or more components or sensors (not explicitly shown). Alternatively, or additionally, the I/O port 108 may communicate with another controller.

The user interface 110 may include a display and a means for receiving user input (e.g., a microphone, a joystick, a satellite dish, a scanner, a printer, a wired and/or wireless device, a keyboard, a pen, a voice input device, a touch input device, a touch-screen device, an interactive display device, a mouse, and/or the like). In some cases, the user interface 110 may be integral to, or a part of, the computing device 100. Alternatively, or additionally, the computing device 100 may be operatively coupled to a remotely located user interface 110 including a display and a means for receiving user input. For example, the remotely located user interface 110 may be a separate display, a portable device, such as, but not limited to a smartphone, tablet computer, laptop computer, etc., or other such device. Additionally, or alternatively, the user interface 110 may further include other components configured to present information to a user such as, for example, a display device, a speaker, a printing device, and/or the like. In some cases, a user interface 110 may be omitted.

The bus 104 represents what may be one or more busses (such as, for example, an address bus, data bus, or combination thereof). Similarly, in embodiments, the computing device 100 may include a number of processing units 102, a number of memory components 106, a number of I/O ports 108, a number of user interface components 110, and/or a number of power supplies 112. Additionally, any number of these components, or combinations thereof, may be distributed and/or duplicated across a number of computing devices.

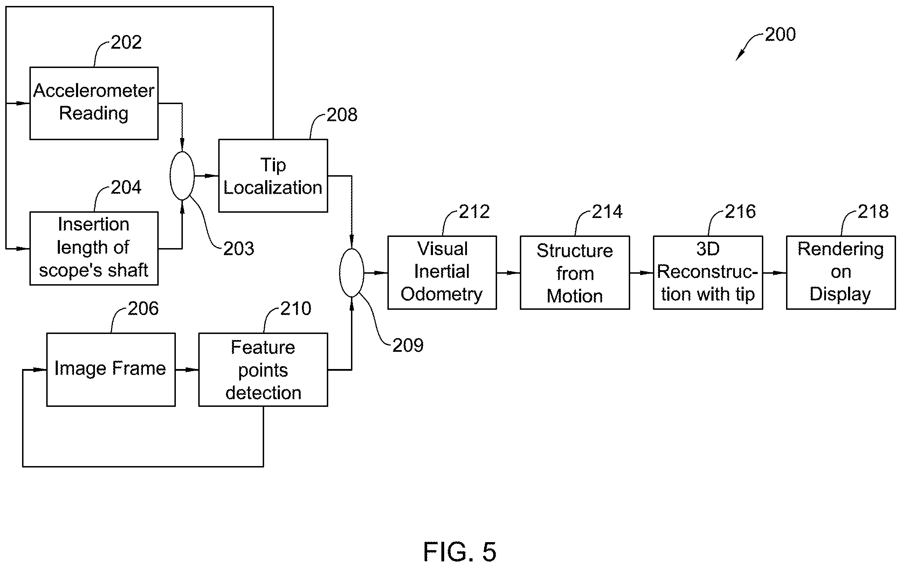

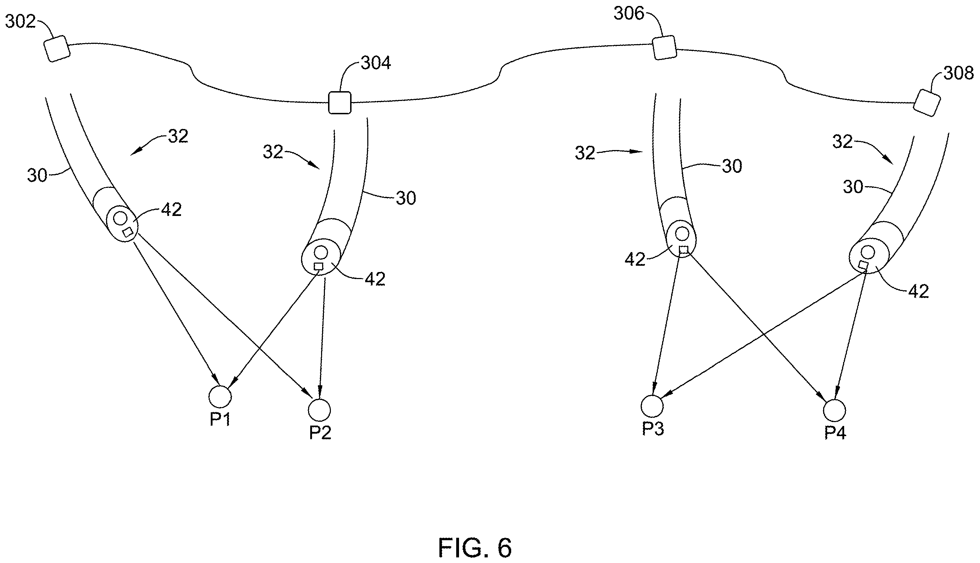

The computing device may receive data from the camera 33, accelerometer 36, and optic sensors 70, 72 to determine and map a location of the distal end region 32 of the elongate shaft 30. FIG. 5 is an illustrative flow chart 200 of a method for determining the location of the distal end region 32 of the elongate shaft 30 and rendering the endoscopic system 20 on a display. FIG. 6 illustrates a schematic representation of the elongate shaft 30 moving through the body and obtaining data. To begin, the computing device 100 may receive an accelerometer reading 202, an elongate shaft insertion length 204, and an image 206. It is contemplated that these three data points 202, 204, 206 may be gathered simultaneously, or at substantially the same time. In some cases, the camera 33, accelerometer 36, and optic sensors 70, 72 may be configured to transmit data at predefined time intervals. Alternatively, or additionally, the camera 33, accelerometer 36, and optic sensors 70, 72 may be configured to transmit data in response to a request from the computing device 100. The camera 33, accelerometer 36, and optic sensors 70, 72 may be configured to collect data at a plurality of tip locations 302, 304, 306, 308 (see, for example, FIG. 6) and combine the data to render a 3D reconstruction of the anatomy with the distal end region 32 superimposed thereon.

The accelerometer 36 may transmit the accelerometer reading 202 as raw count data for the X, Y, and Z axis. The raw count may depend on the measurable range of force (e.g., g-force) and the resolution. For example, the accelerometer 36 may have a range of about 8g with a 14 A/D converter (ADC) resolution. However, a range of less than 8g or more than 8g may be used, as desired. Further, the resolution may be less than 14 ADC or greater than 14 ADC, as desired. The raw count data may be converted to acceleration values in appropriate units, such as meters per second squared (m/s²). The optical sensors 70, 72 may be configured to maintain a count of the demarcations 60 which can be converted into the insertion length 204 of the elongate shaft 30. For example, the optical sensors 70, 72 may be configured to count both distal advancement and proximal retraction of the elongate shaft 30 so that the length of the shaft 30 that is within the patient can be determined at any point in time. The accelerometer reading 202 and the elongate shaft 30 insertion length 204 at any temporal occasion may be combined or merged 203 to localize the distal end region 32 of the elongate shaft, as shown at block 208. This may provide an estimate or approximation of the tip location. It is contemplated that an accelerometer reading 202 and an insertion length 204 may be determined for a plurality of tip locations 302, 304, 306, 308. Fewer than four or more than four tip locations may be used, as desired. For example, steps 202, 204, 203, 208 may be repeated for a plurality of different tip locations.

In some cases, the camera 33 may be configured to transmit live video or still images as the endoscopic system 20 is advanced through the body. It is contemplated that an individual image frame 206 may be analyzed for feature point detection 210. For example, the camera 33 may constantly (i.e., continuously) or intermittently capture image frames 206 from which feature points may be extracted. Feature points P1, P2, P3, P4 (see, for example, FIG. 6) may be anatomical structures within the body. In some cases, features points P1, P2, P3, P4 may be captured in more than one image from different angles or viewpoints. For example, first and second feature points P1, P2 are both captured from a first tip location 302 and a second tip location 304. Similarly, third and fourth feature points P3, P4 are both captured from a third tip location 306 and a fourth tip location 308. This is just one example. Any number of feature points may be used. Further, each image is not limited to two feature points nor is it required for feature points to be present in two images. It is contemplated that feature points from a plurality of different image frames (e.g., which are captured at different tip locations 302, 304, 306, 308) may be combined to generate a point cloud. For example, steps 206, 210 may be repeated for a plurality of different tip locations. The point cloud in turn may be used to generate a view of an anatomical structure. In one example, a 3-D surface of an organ, such as a kidney, or a luminal surface of a body lumen may be generated. Other anatomical structures may be generated as desired.

The computing device 100 may then combine 209 the tip localization data 208 and the feature point data 210 to perform visual inertial odometry 212 to determine an accurate tip location. For example, the position and orientation of the distal end 42 of the elongate shaft 30 may be determined by analyzing the image and the movement of the distal end region 32 (as determined by the insertion length 204 and accelerometer reading). After the tip location is calculated, structure from motion imaging technique 214 may then be applied to calculate the 3D coordinates of the feature's points. For example, the local motion signals (e.g., from the accelerometer), 3D coordinates of the feature points, and/or the two-dimensional images from the camera 33 may be combined to generate a 3D structure or image of the anatomical structure.

The computing device 100 then utilizes the structure from motion analysis 214 to generate a 3D reconstruction 216 of the anatomical structure with the distal end region 32 of the elongate shaft 30. This computing device 100 may then display the rendering on the user interface 110 or other display, as shown at block 218.

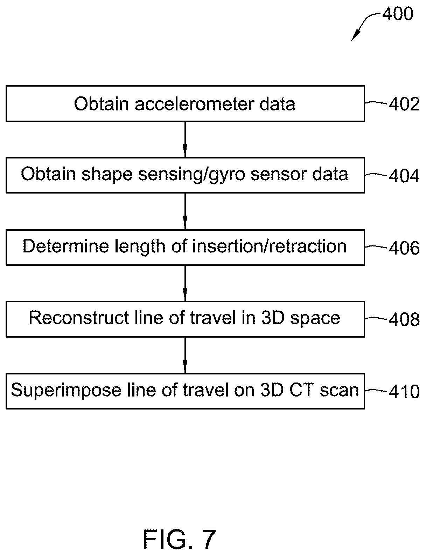

FIG. 7 is a flow chart of another illustrative method 400 for tracking a location of an endoscopic system 20. To begin, the computing device 100 may receive or obtain accelerometer data 402, data from a shape sensing or gyro sensor 404, and an elongate shaft insertion and/or retraction length 406. Shape sensing and/or a gyro sensor may provide angulation information of the distal tip which may be used in 3D reconstruction. Alternatively, or in addition to a shape sensing or gyro sensor 404, a camera mounted on the distal tip of the endoscopic system 20 may be used to supplement the accelerometer data 402 when determining a tip location. As described above, the accelerometer 36 may transmit the accelerometer reading 402 as raw count data for the X, Y, and Z axis. The raw count may depend on the measurable range of force (e.g., g-force) and the resolution. For example, the accelerometer 36 may have a range of about 8g with a 14 A/D converter (ADC) resolution. However, a range of less than 8g or more than 8g may be used, as desired. Further, the resolution may be less than 14 ADC or greater than 14 ADC, as desired. The raw count data may be converted to acceleration values in appropriate units, such as meters per second squared (m/s²). The shaft insertion and/or retraction length 406 may be determined utilizing sensors 70, 72 positioned outside the body. The optical sensors 70, 72 may be configured to maintain a count of the demarcations 60 which can be converted into the insertion length 406 of the elongate shaft 30. For example, the optical sensors 70, 72 may be configured to count both distal advancement and proximal retraction of the elongate shaft 30 so that the length of the shaft 30 that is within the patient can be determined at any point in time.

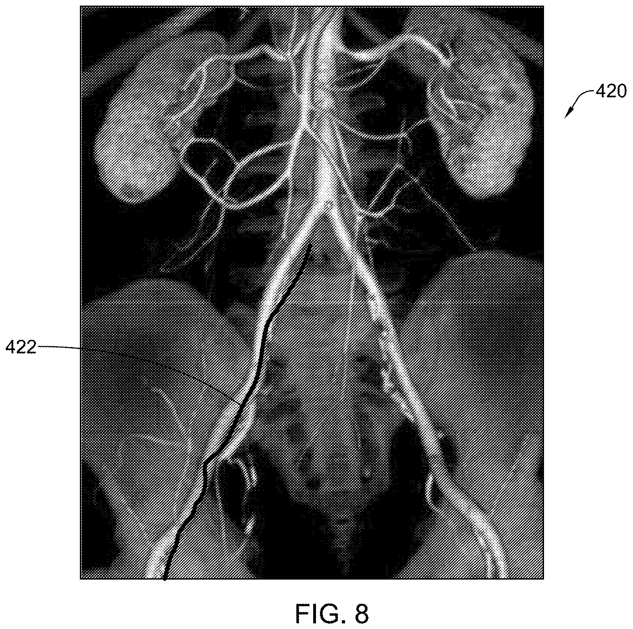

The computing device 100 may be programmed to utilize the accelerometer data 402, the data from a shape sensing or gyro sensor 404 and the shaft insertion and/or retraction length 406 to reconstruct or generate a line of travel of the elongate shaft 30 in 3D space 408. The computing device 100 may then superimpose the line of travel over a 3D CT scan of the anatomy, or other imaging technique, to be displayed on the user interface 110. FIG. 8 illustrates a line of travel 422 of the elongate shaft 30 over a 3D scan 420 of the intrarenal area. It is contemplated that virtual markings may be positioned on the scan 420 to facilitate navigation to a previous position of the elongate shaft. In other cases, virtual markings may be used to help locate kidney stones. Virtual markings or pins may be dropped or placed on the display through actuation of a button on the handle 22 of the endoscopic system 20 or through the user interface 110, as desired.

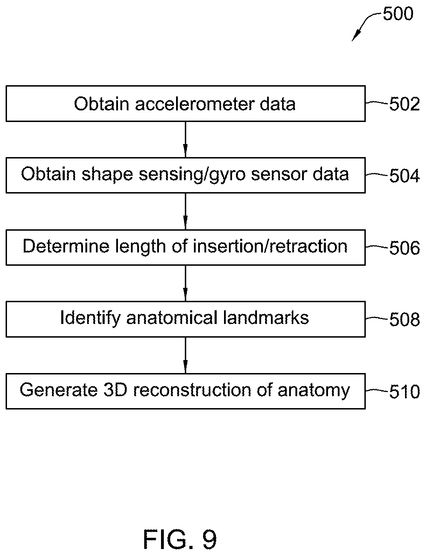

FIG. 9 is a flow chart of another illustrative method 500 for tracking a location of an endoscopic system 20. To begin, the computing device 100 may receive or obtain accelerometer data 502, data from a shape sensing or gyro sensor 504, and an elongate shaft insertion and/or retraction length 506. Shape sensing and/or a gyro sensor may provide angulation information of the distal tip which may be used in 3D reconstruction. Alternatively, or in addition to a shape sensing or gyro sensor 404, a camera mounted on the distal tip of the endoscopic system 20 may be used to supplement the accelerometer data 402 when determining a tip location. As described above, the accelerometer 36 may transmit the accelerometer reading 502 as raw count data for the X, Y, and Z axis. The raw count may depend on the measurable range of force (e.g., g-force) and the resolution. For example, the accelerometer 36 may have a range of about 8g with a 14 A/D converter (ADC) resolution. However, a range of less than 8g or more than 8g may be used, as desired. Further, the resolution may be less than 14 ADC or greater than 14 ADC, as desired. The raw count data may be converted to acceleration values in appropriate units, such as meters per second squared (m/s²). The shaft insertion and/or retraction length 506 may be determined utilizing sensors 70, 72 positioned outside the body. The optical sensors 70, 72 may be configured to maintain a count of the demarcations 60 which can be converted into the insertion length 506 of the elongate shaft 30. For example, the optical sensors 70, 72 may be configured to count both distal advancement and proximal retraction of the elongate shaft 30 so that the length of the shaft 30 that is within the patient can be determined at any point in time.

The computing device 100 may be further configured to identify anatomical landmarks 508 utilizing video data or still images obtained from the camera 33. For example, the computing device 100 may be configured to utilize finger printing techniques and/or optical navigation to obtain a picture of the anatomy. The computing device 100 may be programmed to utilize the accelerometer data 402, the data from a shape sensing or gyro sensor 404 and the shaft insertion and/or retraction length 406, and the anatomical landmarks 410 to reconstruct or generate a 3D reconstruction of the anatomy. It is contemplated that virtual markings may be positioned on the reconstruction to facilitate navigation to a previous position of the elongate shaft. In other cases, virtual markings may be used to help locate kidney stones. Virtual markings or pins may be dropped or placed on the display through actuation of a button on the handle 22 of the endoscopic system 20 or through the user interface 110, as desired.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A system (20) for determining a position of a medical device in a body, the system (20) comprising:
an elongate shaft (30) extending from a proximal end to a distal end;
an accelerometer (36) located in the elongate shaft (30) adjacent to the distal end thereof and configured to provide a direction of movement of a distal tip of the elongate shaft (30) in X, Y, and Z directions;
a plurality of demarcations (60) formed on an outer surface of the elongate shaft (30), the demarcations (60) longitudinally spaced at predefined intervals along a length of the elongate shaft (30);
a first optic sensor (70) and a second optic sensor (72), the first and second optic sensors (70, 72) configured to be positioned adjacent to the body and longitudinally spaced apart and to detect and count the plurality of demarcations (60) as the elongate shaft (30) is advanced into the body; and
a computing device (100) operably coupled to the accelerometer (36), the first optic sensor (70) and the second optic sensor (72), the computing device (100) configured to:
determine an insertion length of the elongate shaft (30) that has been advanced into the body from a reference point based on the detection and counting of the plurality of demarcations (60), and
compute the movement of the medical device based on the insertion length
and the direction of movement of the distal tip provided by the accelerometer (36).

2. The system (20) of claim 1, wherein the first and second optic sensors (70, 72) are configured to detect a direction of travel of the elongate shaft (30).

3. The system (20) of any one of claims 1-2, further comprising a gyro sensor (404; 504).

4. The system (20) of any one of claims 1-3, wherein the accelerometer (36) includes an accelerometer chip embedded in the distal tip of the elongate shaft (30).

5. The system (20) of any one of claims 1-4, further including a handle (22) coupled to a proximal end of the elongate shaft (30).

6. The system (20) of claim 5, further including at least one data transmission cable (56) extending proximally from the accelerometer (36) through the elongate shaft (30) to the handle (22).

7. The system (20) of any one of claims 1-6, wherein the computing device (100) is configured to merge the accelerometer data and the length of the elongate shaft (30) to localize the distal end region of the elongate shaft (30), reconstruct a line of travel of the medical device within the body, and superimpose the reconstructed line of travel over an image of an anatomy of the patient.

8. The system (20) of claim 7, wherein the image of the anatomy is a 3-dimensional (3D) reconstruction of an anatomy adjacent to the distal end region of the elongate shaft (30).

9. The system (20) of claim 7, wherein the image of the anatomy is a 3-dimensional CT scan.

10. The system (20) of any one of claims 1-9, further comprising a camera (33) positioned at the distal end of the elongate shaft (30), wherein the computing device (100) is configured to obtain an image from the camera (33).

11. The system (20) of claim 10, wherein the computing device (100) is configured to detect feature points from the image.

12. The system (20) of claim 11, wherein the computing device (100) is configured to merge the feature points and a localized distal end region of the elongate shaft (30).

13. The system (20) of any one of claims 1-12, wherein the computing device (100) is configured to obtain the data from the accelerometer (36) and determine the length of the elongate shaft (30) inserted into the body with the distal end of the elongate shaft (30) at a same location.

14. The system (20) of any one of claims 1-13, wherein the computing device (100) is configured to obtain the accelerometer data from a plurality of different locations of the distal end of the elongate shaft (30).

15. The system (20) of any one of claims 1-14, wherein the computing device (100) is configured to determining the length of the elongate shaft (30) inserted into the body from a plurality of different locations of the distal end of the elongate shaft (30).

## Patentansprüche

1. System (20) zum Bestimmen einer Position einer medizinischen Vorrichtung in einem Körper, wobei das System (20) umfasst:
einen länglichen Schaft (30), der sich von einem proximalen Ende zu einem distalen Ende erstreckt;
einen Beschleunigungsmesser (36), der sich in dem länglichen Schaft (30) neben dessen distalem Ende befindet und eingerichtet ist, eine Bewegungsrichtung einer distalen Spitze des länglichen Schafts (30) in X-, Y- und Z-Richtung bereitzustellen;
eine Vielzahl von Abgrenzungen (60), die an einer Außenfläche des länglichen Schafts (30) ausgebildet sind, wobei die Abgrenzungen (60) in vordefinierten Abständen entlang einer Länge des länglichen Schafts (30) in Längsrichtung beabstandet sind;
einen ersten optischen Sensor (70) und einen zweiten optischen Sensor (72), wobei der erste und der zweite optische Sensor (70, 72) eingerichtet sind, benachbart zum Körper und in Längsrichtung voneinander beabstandet positioniert zu sein und die Vielzahl von Abgrenzungen (60) zu detektieren und zählen, wenn der längliche Schaft (30) in den Körper vorgeschoben wird; und
eine Rechenvorrichtung (100), die funktionsfähig mit dem Beschleunigungsmesser (36), dem ersten optischen Sensor (70) und dem zweiten optischen Sensor (72) verbunden ist, wobei die Rechenvorrichtung (100) eingerichtet ist zum:
Bestimmen einer Einführlänge des länglichen Schafts (30), die von einem Referenzpunkt aus in den Körper vorgeschoben wurde, auf der Grundlage der Detektion und Zählung der Vielzahl von Abgrenzungen (60), und
Berechnen des Bewegens der medizinischen Vorrichtung auf der Grundlage der Einführlänge und der Bewegungsrichtung der distalen Spitze, die vom Beschleunigungsmesser (36) bereitgestellt wird.

2. System (20) nach Anspruch 1, wobei der erste und der zweite optische Sensor (70, 72) eingerichtet sind, eine Bewegungsrichtung des länglichen Schafts (30) zu detektieren.

3. System (20) nach einem der Ansprüche 1 bis 2, das ferner einen Gyrosensor (404; 504) umfasst.

4. System (20) nach einem der Ansprüche 1 bis 3, wobei der Beschleunigungsmesser (36) einen Beschleunigungsmesserchip umfasst, der in die distale Spitze des länglichen Schafts (30) eingebettet ist.

5. System (20) nach einem der Ansprüche 1 bis 4, das ferner einen Griff (22) umfasst, der mit einem proximalen Ende des länglichen Schafts (30) verbunden ist.

6. System (20) nach Anspruch 5, das ferner mindestens ein Datenübertragungskabel (56) umfasst, das sich proximal vom Beschleunigungsmesser (36) durch den länglichen Schaft (30) zum Griff (22) erstreckt.

7. System (20) nach einem der Ansprüche 1 bis 6, wobei die Rechenvorrichtung (100) eingerichtet ist, die Beschleunigungsmesserdaten und die Länge des länglichen Schafts (30) zusammenzuführen, um den distalen Endbereich des länglichen Schafts (30) zu lokalisieren, eine Bewegungslinie der medizinischen Vorrichtung innerhalb des Körpers zu rekonstruieren und die rekonstruierte Bewegungslinie über ein Bild einer Anatomie des Patienten zu legen.

8. System (20) nach Anspruch 7, wobei das Bild der Anatomie eine dreidimensionale (3D) Rekonstruktion einer Anatomie ist, die an den distalen Endbereich des länglichen Schafts (30) angrenzt.

9. System (20) nach Anspruch 7, wobei das Bild der Anatomie ein dreidimensionaler CT-Scan ist.

10. System (20) nach einem der Ansprüche 1 bis 9, das ferner eine Kamera (33) umfasst, die am distalen Ende des länglichen Schafts (30) positioniert ist, wobei die Rechenvorrichtung (100) eingerichtet ist, ein Bild von der Kamera (33) zu erhalten.

11. System (20) nach Anspruch 10, wobei die Rechenvorrichtung (100) eingerichtet ist, Merkmalspunkte aus dem Bild zu detektieren.

12. System (20) nach Anspruch 11, wobei die Rechenvorrichtung (100) eingerichtet ist, die Merkmalspunkte und einen lokalisierten distalen Endbereich des länglichen Schafts (30) zusammenzuführen.

13. System (20) nach einem der Ansprüche 1 bis 12, wobei die Rechenvorrichtung (100) so eingerichtet ist, die Daten vom Beschleunigungsmesser (36) zu erhalten und die Länge des in den Körper eingeführten länglichen Schafts (30) zu bestimmen, wobei sich das distale Ende des länglichen Schafts (30) an derselben Stelle befindet.

14. System (20) nach einem der Ansprüche 1 bis 13, wobei die Rechenvorrichtung (100) eingerichtet ist, die Beschleunigungsmesserdaten von einer Vielzahl verschiedener Stellen des distalen Endes des länglichen Schafts (30) zu erhalten.

15. System (20) nach einem der Ansprüche 1 bis 14, wobei die Rechenvorrichtung (100) eingerichtet ist, die Länge des in den Körper eingeführten länglichen Schafts (30) anhand einer Vielzahl verschiedener Positionen des distalen Endes des länglichen Schafts (30) zu bestimmen.

## Revendications

1. Système (20) pour déterminer une position d'un dispositif médical dans un corps, le système (20) comprenant :
un arbre allongé (30) qui est étendu depuis une extrémité proximale jusqu'à une extrémité distale ;
un accéléromètre (36) qui est localisé dans l'arbre allongé (30) en une position adjacente à son extrémité distale et qui est configuré pour fournir une direction de mouvement d'une pointe distale de l'arbre allongé (30) dans des directions X, Y et Z ;
une pluralité de démarcations (60) qui sont formées sur une surface externe de l'arbre allongé (30), les démarcations (60) étant espacées longitudinalement selon des intervalles prédéfinis suivant une longueur de l'arbre allongé (30) ;
un premier capteur optique (70) et un second capteur optique (72), les premier et second capteurs optiques (70, 72) étant configurés pour être positionnés en une position adjacente au corps et pour être espacés longitudinalement ainsi que pour détecter et compter la pluralité de démarcations (60) lorsque l'arbre allongé (30) est avancé à l'intérieur du corps ; et
un dispositif informatique (100) qui est couplé de manière opérationnelle à l'accéléromètre (36), au premier capteur optique (70) et au second capteur optique (72), le dispositif informatique (100) étant configuré pour :
déterminer une longueur d'insertion de l'arbre allongé (30) qui a été avancé à l'intérieur du corps par rapport à un point de référence sur la base de la détection et du comptage de la pluralité de démarcations (60) ; et
calculer le mouvement du dispositif médical sur la base de la longueur d'insertion et de la direction de mouvement de la pointe distale fournie par l'accéléromètre (36).

2. Système (20) selon la revendication 1, dans lequel les premier et second capteurs optiques (70, 72) sont configurés pour détecter une direction de déplacement de l'arbre allongé (30).

3. Système (20) selon l'une quelconque des revendications 1 et 2, comprenant en outre un capteur gyroscopique (404 ; 504).

4. Système (20) selon l'une quelconque des revendications 1 à 3, dans lequel l'accéléromètre (36) inclut une puce d'accéléromètre qui est intégrée dans la pointe distale de l'arbre allongé (30).

5. Système (20) selon l'une quelconque des revendications 1 à 4, incluant en outre une poignée (22) qui est couplée à une extrémité proximale de l'arbre allongé (30).

6. Système (20) selon la revendication 5, incluant en outre au moins un câble de transmission de données (56) qui est étendu de façon proximale depuis l'accéléromètre (36) au travers de l'arbre allongé (30) jusqu'à la poignée (22).

7. Système (20) selon l'une quelconque des revendications 1 à 6, dans lequel le dispositif informatique (100) est configuré pour fusionner les données d'accéléromètre et la longueur de l'arbre longitudinal (30) afin de localiser la région d'extrémité distale de l'arbre allongé (30), de reconstruire une ligne de déplacement du dispositif médical à l'intérieur du corps et de superposer la ligne de déplacement reconstruite sur une image d'une anatomie du patient.

8. Système (20) selon la revendication 7, dans lequel l'image de l'anatomie est une reconstruction tridimensionnelle (3D) d'une anatomie en une position adjacente à la région d'extrémité distale de l'arbre allongé (30).

9. Système (20) selon la revendication 7, dans lequel l'image de l'anatomie est une image de tomodensitométrie tridimensionnelle.

10. Système (20) selon l'une quelconque des revendications 1 à 9, comprenant en outre une caméra (33) qui est positionnée au niveau de l'extrémité distale de l'arbre allongé (30), dans lequel le dispositif informatique (100) est configuré pour obtenir une image à partir de la caméra (33).

11. Système (20) selon la revendication 10, dans lequel le dispositif informatique (100) est configuré pour détecter des points de caractéristique à partir de l'image.

12. Système (20) selon la revendication 11, dans lequel le dispositif informatique (100) est configuré pour fusionner les points de caractéristique et une région d'extrémité distale localisée de l'arbre allongé (30).

13. Système (20) selon l'une quelconque des revendications 1 à 12, dans lequel le dispositif (100) est configuré pour obtenir les données en provenance de l'accéléromètre (36) et pour déterminer la longueur de l'arbre allongé (30) qui est inséré à l'intérieur du corps tandis que l'extrémité distale de l'arbre allongé (30) est au niveau d'une même localisation.

14. Système (20) selon l'une quelconque des revendications 1 à 13, dans lequel le dispositif informatique (100) est configuré pour obtenir les données d'accéléromètre à partir d'une pluralité de différentes localisations de l'extrémité distale de l'arbre allongé (30).

15. Système (20) selon l'une quelconque des revendications 1 à 14, dans lequel le dispositif informatique (100) est configuré pour déterminer la longueur de l'arbre allongé (30) qui est inséré à l'intérieur du corps à partir d'une pluralité de différentes localisations de l'extrémité distale de l'arbre allongé (30).
